# EUROPEAN PATENT SPECIFICATION

(11) **EP 3 615 149 B1**
(45) Date of publication and mention of the grant of the patent: **10.01.2024**
(21) Application number: 18790404.0
(22) Date of filing: 24.04.2018
(51) Int. Cl.: A61Q 11/00, A61C 9/00, A61B 5/00, A61C 19/04, G06T 7/00

(54) **METHODS OF MONITORING WHITE SPOTS ON TEETH**
VERFAHREN ZUR ÜBERWACHUNG VON WEISSEN FLECKEN AUF ZÄHNEN
PROCÉDÉS DE SURVEILLANCE DE TACHES BLANCHES SUR LES DENTS

(30) Priority: 24.04.2017 US 201762489366 P
(43) Date of publication of application: 04.03.2020
(73) Proprietor: Colgate-Palmolive Company, New York, NY 10022 (US)
(72) Inventor: KILPATRICK-LIVERMAN, LaTonya, Piscataway, NJ 08854 (US); QIU, Jianhong, 909 River Road Piscataway, NJ 08854 (US); SANTARPIA III, Ralph Peter, 909 River Road Piscataway, NJ 08854 (US)
(74) Representative: Wibbelmann, Jobst
(86) International application number: PCT/US2018/029134
(87) International publication number: WO 2018/200520

(56) References cited:
- US-A1- 2004 202 356
- US-A1- 2008 056 551
- US-A1- 2010 165 089
- US-A1- 2010 322 490
- US-A1- 2011 085 713
- US-A1- 2012 148 986
- US-A1- 2016 307 323
- KOUTSOURI GEORGIA D ET AL: "Detection of occlusal caries based on digital image processing", 13TH IEEE INTERNATIONAL CONFERENCE ON BIOINFORMATICS AND BIOENGINEERING, IEEE, 10 November 2013 (2013-11-10), pages 1-4, XP032541308, DOI: 10.1109/BIBE.2013.6701708 [retrieved on 2014-01-02]

## Description

### BACKGROUND

Dental enamel is a thin, hard layer of calcified material that covers the crown of teeth. The major mineral component of dental enamel is hydroxyapatite, a crystalline form of calcium phosphate. Poorly mineralized regions beneath, within or adjacent to intact enamel often appear as white spots on the teeth because such poorly mineralized regions are porous and reflect light differently from enamel. In most cases, white spots are the result of demineralization, for example from acids produced by cariogenic bacteria, which break down the hydroxyapatite into calcium and phosphate ions, leading to porous regions within the enamel. Other common causes of poor mineralization and associated white spot lesions include xerostomia, trauma, and arrested decay that has only partially remineralized around fixed orthodontic appliances, which may provide shelter for bacteria or interfere with normal remineralization. Conditions that interfere with tooth development, such as fluorosis, hypomineralization/hypomaturation, mineral deficiencies, and hypoplasia, can also cause white spot lesions.

The diagnosis and evaluation of white spots is typically performed simply by looking at the teeth. The development of the white spots and/or the efficacy of treatments to remove white spots typically judged simply by looking at "before" and "after" pictures. Such evaluation is subjective and qualitative. The results depend to a large extent on the person making the assessment and the lighting conditions when the assessment is made.

It is possible to measure and compare grayscale values of teeth in a photograph, but the contrast between the white spot and the surrounding white enamel using conventional grayscale values is generally inadequate to provide a degree of distinction sufficient to measure fine changes or differences in the white spots and is subject to distortion by the lighting conditions. US 2012/0148986 A1 discloses a method for identification of caries, executed at least in part on data processing hardware, obtains an original image that has a plurality of colour channels and generates an adjusted image by adjusting intensity values of the original digital image to a range between the minimum value and a maximum value. US 2010/0165089 A1 discloses an apparatus for imaging a tooth having a light source with a first spectral range and a second spectral range.

There is a need for improved methods of quantifying the severity of white spot lesions on the teeth, to allow more accurate assessment and monitoring of the white spots.

### BRIEF SUMMARY

The present inventors have developed a new method of quantifying white spot lesions on the teeth as defined in the claims.

Color digital images are made of pixels, a pixel being the smallest controllable element of a digital picture. Pixels in a color digital image are made of combinations of primary colors represented by a series of code. Grayscale is a measure of intensity. A "channel" in this context is the grayscale image of the same subject as a color image, made of just one of these primary colors. For instance, an image from a standard digital camera will have a red, green and blue channel. CMYK images for printing have four channels for cyan, magenta, yellow and black ink. The digital code for each pixel therefore provides an address (where in the image it is located) and a grayscale (intensity value) for each channel. The blend of the intensity of each channel in each pixel and the blend of pixels in the picture all combine to provide the image that we see.

This disclosure provides methods of quantifying the severity and monitoring the progression of white spot lesions on a tooth by quantifying the grayscale difference between the white spot lesions and the surrounding healthy enamel in the blue channel of a digital color photographic image of the tooth as defined in the claims. Focusing on the contrast in the blue spectrum highlights the differences between the white spot areas and the sound enamel (which is naturally slightly yellow and therefore absorbs blue light) and digitally filters out much of the variability generated by different lighting in different environments, providing a more accurate and consistent measure of the severity of the white spot relative to the adjacent enamel. The blue channel is the channel corresponding to a color with a wavelength in the range of 450-520nm, e.g., a color corresponding to about 450-495nm, e.g. about 470nm. The disclosure thus provides a method of quantifying the white spot lesions on a tooth comprising quantifying the grayscale difference between the white spot lesions and the surrounding healthy enamel in the blue channel of a digital color photographic image of the tooth; wherein the method comprises:
a) drying the tooth to be evaluated;
b) obtaining a digital color photographic image of the tooth; and
d) measuring the difference between the grayscale value of at least one pixel in an area of the image depicting at least a portion of the white spot (e.g. of the pixel having high or the highest grayscale value in such area) and the grayscale value of at least one pixel in an area of the image depicting at least a portion of the adjacent healthy enamel (e.g. of the pixel having low or the lowest grayscale value in such area), wherein the greyscale values are measured only in the blue channel of the image.

The difference between the the grayscale value of the pixel in the area depicting the white spot and the grayscale value of the pixel in the area depicting the adjacent healthy enamel is a measure of the contrast between the white spot and the new adjacent enamel. The greater the contrast, the more noticeable and/or severe the white spot is. If the contrast increases over time, that indicates the white spot is getting worse. If the contrast reduces, that indicates the severity of the white spot is reduced, at least from an aesthetic perspective.

For example, the white spot repair efficacy of a proposed treatment can be calculated as follows:
1) Before treatment (baseline): Contrast 1 = blue channel grayvalue of white spot (highest value) - blue channel grayvalue of adjacent area of sound enamel (lowest value);
2) After treatment: Contrast 2 = blue channel grayvalue of white spot (highest value) - blue channel greyvalue of adjacent area of sound enamel (lowest value) (Same areas are measured for baseline and post treatment);
3) White spot repair efficacy of the treatment = Contrast 2 - Contrast 1
Negative values represent decreased contrast, corresponding to white spot lesion repair that includes improved aesthetics, e.g., the white spot color is closer to the color of the surrounding enamel and therefore less visible.

Not claimed is a method of monitoring the progress of a white spot condition on the teeth of a patient comprising quantifying the severity of white spots on teeth over time, wherein a reduction in the difference between the grayscale value of at least one pixel in the area depicting at least a portion of the white spot and the grayscale value of at least one pixel in the area depicting at least a portion of the adjacent healthy enamel indicates repair of the white spot.

Further areas of applicability of the present invention will become apparent from the detailed description provided hereinafter.

### DETAILED DESCRIPTION

Digital cameras and image processing software are widely available, and virtually all image processing programs permit shifting of color balance, which would enable them to be used for the methods of the instant disclosure.

For example, ImageJ is a public domain, Java-based image processing program developed at the National Institutes of Health, which can be downloaded free at https://imagej.nih.gov/ij/. Using ImageJ, the operations can be carried out as follows:
1. Take baseline and post treatment images on white spot and adjacent sound area (same area(s) for baseline and post-treatment) using SpectroShade.
2. Open the image files being compared at once so that they all can be seen on the screen together.
3. Click on the window of interest (e.g baseline at first)
4. Click Image -> Color >Split Channels, and it will split the image into three channels - green, red, and blue.
5. The blue channel shows the best binary image. Delete the other two.
6. Select an area of the baseline image to be analyzed. Use the rectangle tool to drag a rectangle across the desired area - it should contain part of the white spot and part of the normal crown of the tooth. The purpose of this is to compare the contrast of the white spot versus the normal part, a.k.a. how white is the white spot compared to the rest of the tooth?
4. After the area is chosen, retrieve the minimum and maximum values (darkest and lightest):
   4-a. Click Analyze □ Plot Profile and a window will pop up with a plot of the contrast by pixel.
   4-b. Click the List button to see a list of values
   4-c. Go through the list, record the lowest and highest values.
5. Calculate the difference between the minimum and maximum and record. This difference is the Contrast 1(between white spot and sound area).
6. Repeat for the post-treatment image (step 3-5). Make sure same area is analyzed for baseline and post-treatment. Then Contrast 2 is obtained from post-treatment image.
7. The contrast differences between baseline and post-treatment (contrast 2 - contrast 1) reflect the effect of the treatment. More negative (less contract compared to baseline) means better white spot lesion repair (removal).

The following examples illustrate methods for use in accordance with the present disclosure. The exemplified methods are illustrative and do not limit the scope of the disclosure.

### EXAMPLES

### Example 1 - Measuring White Spot Reduction

Human extracted teeth with white spot(s) are gently brush cleaned and also screened by dentist. The screened teeth are then stored in deionized water at ca. 4°C until the treatments are started.

The stored teeth are taken out, slightly dried with paper towel before the base line images of the white spots and adjacent sound areas are taken. The contrast values between the white spot and adjacent sound areas are quantified using ImageJ.

The teeth are then soaked in one of three test solutions or control mouthwash for 30 minutes, 15ml for each tooth, at 37°C, while being spun at 100 rpm. The teeth are then rinsed for 5 minutes with deionized water at 400 rpm. The teeth are then paper towel dried, and the deionized water rinse is repeated. The treated teeth are then incubated in artificial saliva overnight at 37° C.

This treatment, washing and incubation is repeated daily for nine more days, for a total of 10 days of treatment. After the 10 days of treatment is completed, the teeth are rinsed twice with deionized water as described.

In order to avoid false repair readings, e.g., decreased contrast from loosely deposited mineral (from artificial saliva) on tooth surface, the teeth are brushed using medium toothbrush after all treatment/incubation. The teeth are each brushed under tap water, continuously and evenly at all angles for two minutes, rinsed under tap water, and placed onto a paper towel to dry.

Final images of the treated teeth, after brushing, are taken after the teeth are slightly dried with the paper towel. The contrast between the white spot area and the adjacent sound enamel is again quantified. Reduction in contrast (negative value) indicates degree of repair of the white spot. The results are set forth in Table 2:

**Table 2**

| **Material** | **Contrast change** |
|---|---|
| 1 | -21.4 |
| 2 | -18.6 |
| 3 | -19.5 |
| Control | 0.76 |

This data shows that the three novel treatment solutions reduced the white spot lesions, while the control treatment had little effect on the white spots.

## Claims

1. A method of quantifying white spot lesions on a tooth comprising quantifying the grayscale difference between the white spot lesions and the surrounding healthy enamel in the blue channel of a digital color photographic image of the tooth; wherein the method comprises:
a) drying the tooth to be evaluated;
b) obtaining a digital color photographic image of the tooth; and
c) measuring the difference between the grayscale value of at least one pixel in an area of the image depicting at least a portion of the white spot and the grayscale value of at least one pixel in an area of the image depicting at least a portion of the adjacent healthy enamel, wherein the greyscale values are measured only in the blue channel of the image.

2. The method of claim 1 wherein the digital color photographic image of the tooth surface in digital form comprises red, green and blue channel information.

3. The method of claim 1 or 2 wherein the blue channel of the digital color photographic image is a channel that provides a color having a wavelength of in the range of 450-520nm.

4. The method of any of claims 1-3 wherein the at least one pixel in the area depicting at least a portion of the white spot is a pixel having high grayscale value in such area, and the least one pixel in the area depicting at least a portion of the adjacent healthy enamel is a pixel having low grayscale value in such area.

5. The method of any of claims 1-4 wherein the amount of difference between the grayscale value of the pixel in the area depicting the white spot and the grayscale value of the pixel in the area depicting the adjacent healthy enamel corresponds to the severity of the white spot.

## Patentansprüche

1. Verfahren zur Quantifizierung von Weißfleckläsionen (Engl.: white spot lesions) auf einem Zahn, umfassend die Quantifizierung der Graustufendifferenz zwischen den Weißfleckläsionen und dem umgebenden gesunden Zahnschmelz in dem blauen Kanal eines digitalen farbfotographischen Bildes des Zahns; wobei das Verfahren umfasst:
a) Trocknen des zu evaluierenden Zahns;
b) Erhalten eines digitalen farbfotographischen Bildes des Zahns; und
c) Messen der Differenz zwischen dem Graustufenwert von mindestens einem Pixel in einem Bereich des Bildes, der mindestens einen Teil des weißen Flecks darstellt, und dem Graustufenwert von mindestens einem Pixel in einem Bereich des Bildes, der mindestens einen Teil des angrenzenden gesunden Zahnschmelzes darstellt, wobei die Graustufenwerte nur im blauen Kanal des Bildes gemessen werden.

2. Das Verfahren nach Anspruch 1, wobei das digitale farbfotographische Bild der Zahnoberfläche in digitaler Form Rot-, Grün- und Blaukanalinformationen umfasst.

3. Das Verfahren nach Anspruch 1 oder 2, wobei der blaue Kanal des digitalen farbfotografischen Bildes ein Kanal ist, der eine Farbe mit einer Wellenlänge im Bereich von 450-520nm bereitstellt.

4. Das Verfahren nach einem der Ansprüche 1 bis 3, wobei das mindestens eine Pixel in dem Bereich, der mindestens einen Teil des weißen Flecks darstellt, ein Pixel mit hohem Graustufenwert in einem solchen Bereich ist, und das mindestens eine Pixel in dem Bereich, der mindestens einen Teil des angrenzenden gesunden Zahnschmelzes darstellt, ein Pixel mit niedrigem Graustufenwert in einem solchen Bereich ist.

5. Das Verfahren nach einem der Ansprüche 1-4, wobei der Betrag der Differenz zwischen dem Graustufenwert des Pixels in dem Bereich, der den weißen Fleck darstellt, und dem Graustufenwert des Pixels in dem Bereich, der den angrenzenden gesunden Zahnschmelz darstellt, dem Schweregrad des weißen Flecks entspricht.

## Revendications

1. Procédé de quantification de lésions ponctuelles blanches sur une dent comprenant la quantification de la différence en échelle de gris entre les lésions ponctuelles blanches et l'émail sain environnant dans le canal bleu d'une image photographique couleur numérique de la dent ; dans lequel le procédé comprend :
a) le séchage de la dent à évaluer ;
b) l'obtention d'une image photographique couleur numérique de la dent ; et
c) la mesure de la différence entre la valeur de niveau de gris d'au moins un pixel dans une zone de l'image représentant au moins une partie de la tache blanche et la valeur de niveau de gris d'au moins un pixel dans une zone de l'image représentant au moins une partie de l'émail sain adjacent, les valeurs de niveaux de gris étant mesurées uniquement dans le canal bleu de l'image.

2. Procédé selon la revendication 1, dans lequel l'image photographique couleur numérique de la surface de la dent sous forme numérique comprend des informations de canal rouge, vert et bleu.

3. Procédé selon la revendication 1 ou 2, dans lequel le canal bleu de l'image photographique couleur numérique est un canal qui fournit une couleur ayant une longueur d'onde dans la plage de 450 à 520 nm.

4. Procédé selon l'une quelconque des revendications 1 à 3, dans lequel l'au moins un pixel dans la zone représentant au moins une partie de la tache blanche est un pixel ayant une valeur de niveau de gris élevée dans une telle zone, et l'au moins un pixel dans la zone représentant au moins une partie de l'émail sain adjacent est un pixel ayant une faible valeur de niveau de gris dans cette zone.

5. Procédé selon l'une quelconque des revendications 1 à 4, dans lequel la quantité de différence entre la valeur de niveau de gris du pixel dans la zone représentant la tache blanche et la valeur de niveau de gris du pixel dans la zone représentant l'émail sain adjacent correspond à la gravité de la tache blanche.
